# EUROPEAN PATENT SPECIFICATION

(11) **EP 1 490 067 B1**
(45) Date of publication and mention of the grant of the patent: **17.09.2008**
(21) Application number: 03743001.4
(22) Date of filing: 26.02.2003
(51) Int. Cl.: A61K 31/505, A61P 25/30

(54) **USE OF TYROSINE KINASE INHIBITORS FOR TREATING SUBSTANCE USE DISORDERS**
VERWENDUNG VON TYROSINKINASE-HEMMERN ZUR BEHANDLUNG VON ERKRANKUNGEN IM ZUSAMMENHANG MIT SUBSTANZGEBRAUCH
UTILISATION D'INHIBITEURS DE TYROSINE KINASE POUR TRAITER DES TROUBLES LIES A L'UTILISATION DE SUBSTANCES

(30) Priority: 27.02.2002 US 359651 P
(43) Date of publication of application: 29.12.2004
(73) Proprietor: AB Science, 75008 Paris (FR)
(72) Inventor: MOUSSY, Alain, F-75006 Paris (FR); KINET, Jean-Pierre, Lexington, MA 02173 (US)
(74) Representative: Warcoin, Jacques
(86) International application number: PCT/IB2003/001071
(87) International publication number: WO 2003/072106

(56) References cited:
- EP-A- 0 564 409
- LONGLEY B J ET AL: "NEW APPROACHES TO THERAPY FOR MASTOCYTOSIS A CASE FOR TREATMENT WITH KIT KINASE INHIBITORS" HEMATOLOGY - ONCOLOGY CLINICS OF NORTH AMERICA, W.B. SAUNDERS, US, vol. 14, no. 3, June 2000 (2000-06), pages 689-695, XP008010967 ISSN: 0889-8588

## Description

The present invention relates to a use for treating substance use disorders, more particularly drug addiction, drug abuse, drug habituation, drug dependence, withdrawal syndrome and overdose, comprising administering a compound capable of depleting mast cells to a human in need of such treatment. Such compounds are tyrosine kinase inhibitors and more particularly non-toxic, selective and potent c-kit inhibitors. Preferably, said inhibitor is unable to promote death of IL-3 dependent cells cultured in presence of IL-3.

Drug dependence is the result of a phenomenon called tolerance, which is the need to increase the dose of the drug to maintain its full effect, and of physical dependence, which is the habituation of the body to a drug. When the intake of a drug is discontinued, individual may experience unpleasant withdrawal syndrome. This syndrome is difficult to qualify or quantify but it can be illustrated by a strong feeling of unmet satisfaction. This episode has been described by former drug addicted individuals as "a strong scream and complaint emanating from the body". This shows the seriousness and the difficulties encountered by these individuals. In addition, it must be emphasized that drug addiction is accompanied with or may follow psychiatric disorders such as anxiety, depression, and schizophrenia.

We can classify two types of drugs leading to dependence :
- Drugs such as cocaine, marijuana, amphetamine, and hallucinogens are responsible for psychologic dependence.
- Other drugs such as heroin, alcohol and nicotine are more prone to physical dependence but one must not rule out psychologic dependence as well.

Of course, any drug that acts on the CNS may involved a risk of dependence. For example, it is well known that one of the side effect of benzodiazepine derivatives is dependence. In animal models, it has been observed that administration of drugs such as opioids, cocaine, amphetamine, nicotine, and benzodiazepines is associated with enhanced dopaminergic transmission. The problem is that the increased level of DA may be followed by a down regulation of DA receptors. This might explain in part the observed withdrawal symptoms that are sometimes associated with depression, mood disorders, insomnia...and other unwanted dependence disorders.

Drug addiction may be responsible for or arise from job pressure or a familial problems resulting in anxiety or depression. At the extreme of the spectrum, is can result in hospitalization for overdose, withdrawal episodes and associated substance use disorders.

Finally, statistics show that anxiolytics such as benzodiazepines are more and more consumed in western countries, for example in France. Therefore, it is urgent to find solutions to prevent and manage drug dependence and withdrawal symptoms. The socioeconomic consequences of reliable solutions will have a huge impact in modem societies since addiction is often accompanied not only with susceptibility to HIV infection and hepatitis but it is would also have a positive socioeconomic impact.

Consequently, research programs aimed at developing compounds capable of alleviating drug dependence and withdrawal symptoms must be encouraged and considered as a top priority.

Substance abuse and drug addiction introduce changes in neurotransmitter synthesis, storage, release, or in the number and affinity of receptors. This can affect neurotransmission and cause drug dependence and withdrawal symptoms.

Among neurotransmitters, we can cite :
- glutamate and aspartate, which are the major excitatory neurotransmitters, whereas aminobutyric acid (GABA) is the major inhibitory neurotransmitter in the brain,
- dopamin (DA) which was observed in depressed patients (Kapur S. et al., 1992, Biol. Psychiat. 32, 1-17),
- GABA which was also shown to be involved in the physiopathology of depression (Lloyd K.G. et al., 1989, Prog. Neuro-Psychopharmacol. Biol. Psychiat. 13, 341-351),
- serotonin (5-HT) (Biegon A., 1990, Ann. NY Acad. Sci. 600, 427-431),
- the well known acetylcholine,
- norepinephrine which interacts with adrenergic receptors and which is regulated by tyrosine hydroxylase and monoamine oxidase,
- endorphins which are polypeptides that activate many central neurons and interact with opioid receptors,
- and others neurotransmitters such as enkephalins, dynorphins, histamine, vasopressin, vasoactive intestinal peptide, carnosine, bradykinin, cholecystokinin, bombesin, somatostatin, corticotropin releasing factor, neurotensin, and adenosine.

As mentioned above, any imbalance in these neurotransmitters or any deregulation of associated receptors due to drug intake may lead to the development of drug dependence and withdrawal symptoms.

But, as of today, there is no treatment providing relief and help to individuals to withdraw from their addiction.

Against all odds, we identified that mast cells are involved in or contribute to drug dependence and withdrawal symptoms.

Mast cells (MC) are tissue elements derived from a particular subset of hematopoietic stem cells that express CD34, c-kit and CD13 antigens (Kirshenbaum et al, Blood. 94: 2333-2342, 1999 and Ishizaka et al, Curr Opin Immunol. 5: 937-43, 1993). Immature MC progenitors circulate in the bloodstream and differentiate in tissues. These differentiation and proliferation processes are under the influence of cytokines, one of utmost importance being Stem Cell Factor (SCF), also termed Kit ligand (KL), Steel factor (SL) or Mast Cell Growth Factor (MCGF). SCF receptor is encoded by the protooncogene c-kit, that belongs to type III receptor tyrosine kinase subfamily (Boissan and Arock, J Leukoc Biol. 67: 135-48, 2000). This receptor is also expressed on others hematopoietic or non hematopoietic cells. Ligation of c-kit receptor by SCF induces its dimerization followed by its transphosphorylation, leading to the recruitement and activation of various intracytoplasmic substrates. These activated substrates induce multiple intracellular signaling pathways responsible for cell proliferation and activation (Boissan and Arock, 2000). Mast cells are characterized by their heterogeneity, not only regarding tissue location and structure but also at the functional and histochemical levels (Aldenborg and Enerback., Histochem. J. 26: 587-96, 1994 ; Bradding et al. J Immunol. 155: 297-307, 1995 ; Irani et al, J Immunol. 147: 247-53, 1991 ; Miller et al, Curr Opin Immunol. 1: 637-42, 1989 and Welle et al, J Leukoc Biol. 61: 233-45, 1997).

Here, it is postulated that the activation of mast cells by different drugs, especially salicylic derivatives, morphine derivatives, opioids, heroin, amphetamines, alcohol, nicotine, analgesics, anesthetics, and anxiolytics results in the degranulation of mast cells, which participate in the exacerbation of the chemical imbalance responsible for drug habituation and withdrawal syndrome.

Indeed, once activated, mast cells release the content of their granules at proximity of neurons which further stimulate neurons and participate to the feeling of satisfaction. Mast cells involved in the response to such stimulus can be brain mast cells but also other mast cells releasing the content of their granules in the blood stream that ultimately reach sensory, motor or brain neurons. Brain mast cells staining is CTMC staining-like but they show the secretory pattern of MMC, implying that they constitute a particular subset of mast cells presenting specificities.

In addition, following mast cells activation, released granules liberate various factors capable of modulating and altering neurotransmission. Among such factors, we can cite morphine which is bound or stored in mast cells granules. This was shown in morphine perfused dogs by Akcasu A. et al, Int J Clin Pharmacol Ther Toxicol 1985 Jan;23(1):33-7.
Thomas PS et al, Am J Physiol 1992 Jul ; 263 : 67-72 also observed that tobacco smoke induces the release of mediators from canine mast cells and modulates prostaglandin production leading to asthma.

Here, we postulate that mast cells exacerbate in paracrine manner the deregulation of neurotransmission. For example, modulation of neurotransmitters such as serotonin by drug intake activates mast cells, which in turn release the content of their granules, further contributing to the chemical imbalance in the brain leading to dependence disorders. Other mediators released by mast cells can be categorized into vasoactive, nociceptive, proinflammatory and other neurotransmitters. Taken together, these factors are able to induce great disturbance in the activity of neurons, whether they are sensory, motor, or CNS neurons.

We even go further in postulating that mast cells may constitute a reservoir of drugs and that activation of mast cells leads to the release of such drugs such as morphine and other substances, such as histamine for example, that contribute to the prolonged synaptic plasticity engaged initially by the drugs.

We also observed that patients afflicted with mastocytosis are more incline to develop substance use disorders than the normal population. This can be explained by the presence of activating mutations in the c-kit receptor, which induce degranulation of mast cells and a burst of factors contributing to chemical imbalance and neurotransmission alteration.

As a consequence, the present invention proposes to deplete mast cells using compounds that are substantially specific to mast cells. In this regard, tyrosine kinase inhibitors and more particularly c-kit specific kinase inhibitors are proposed to inhibit mast cell proliferation, survival and activation. Indeed, once mast cells are removed, no exacerbation or prolonged neural excitation will take place so that drug dependence is alleviated. In addition, removing mast cells is also of interest for preventing death due to overdose. Indeed, adventitial mast cells have been suggested to potentiate atherosclerosis and vasospasm, thrombosis and premature sudden death in long-term cocaine abusers (Kolodgie FD et al, J Am Coll Cardiol 1991 Jun; 17(7):1553-60).

A new route for treating drug dependence is provided, which consists of destroying mast cells involved in and contributing to the physical and psychological dependence. It has been found that tyrosine kinase inhibitors and more particularly c-kit inhibitors are especially suited to reach this goal.

### Description

The present invention relates to a use for treating substance use disorders comprising administering a compound capable of depleting mast cells to a human in need of such treatment.

Said compounds are selected from N-phenyl-2-pyrimidine-amine derivatives corresponding to formula II : 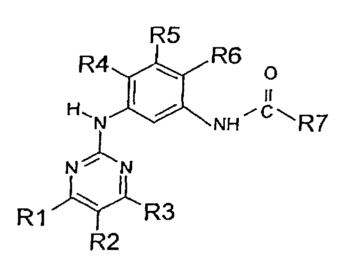 Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function.
Preferably, R7 is the following group :

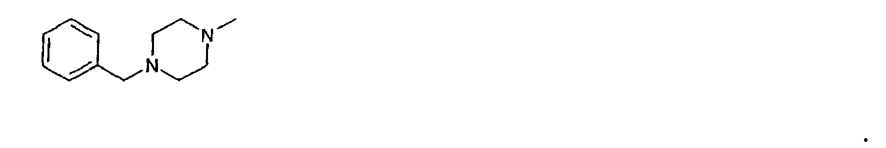

Among these compounds, the preferred are defined as follows:
R1 is a heterocyclic group, especially a pyridyl group,
R2 and R3 are H,
R4 is a C1-C3 alkyl, especially a methyl group,
R5 and R6 are H,
and R7 is a phenyl group bearing at least one substituent, which in turn possesses at least one basic site, such as an amino function, for example the group :

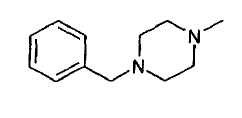

Therefore, in a preferred embodiment, the invention relates to a method for treating substance use disorders comprising the administration of an effective amount of the compound known in the art as CGP57148B:
4-(4-méhylpipérazine-1-ylméthyl)-N-[4-méthyl-3-(4-pyridine-3-yl)pyrimidine-2 ylamino)phényl]-benzamide corresponding to the following formula :

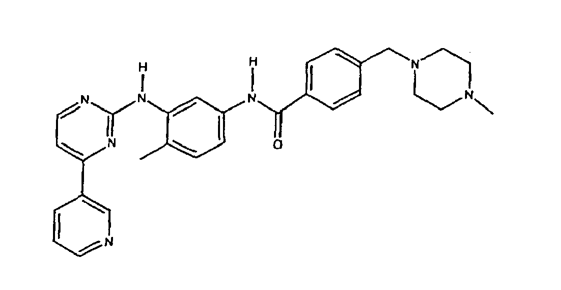

The preparation of this compound is described in example 21 of EP 564 409 and the β-form, which is particularly useful is described in WO 99/03854.

The substance use disorders as referred herein include but are not limited to drug addiction, drug abuse, drug habituation, drug dependence, withdrawal syndrome and overdose.

Therefore, in a preferred embodiment, the method of the invention is applicable to the treatment or prevention of drug addiction.

In another preferred embodiment, the method of the invention is applicable to the treatment of drug abuse.

In another preferred embodiment, the method of the invention is applicable to the treatment or prevention of drug habituation.

In another preferred embodiment, the method of the invention is applicable to the treatment or prevention of drug dependence.

In another preferred embodiment, the method of the invention is applicable to the treatment or prevention of withdrawal syndrome and drug craving.

In another preferred embodiment, the method of the invention is applicable to the treatment or prevention of overdose.

Among drugs that are particularly addictive, we can cite alcohol, nicotine, opioids, cocaine, heroin, anxiolytics and hypnotics such as benzodiazepine, methaqualone and barbiturates, cannabinoids (tetrahydrocannabinol, cannabigerol, cannabinol cannabichromene, cannabidiol, cannabinoid acids), amphetamine such ecstasy, hallucinogen such as LSD, phencyclidine (PCP), mescaline, volatile solvent and volatile nitrites.

Therefore, the invention embraces the use of the compounds defined above to manufacture a medicament for treating substance use disorders such as drug addiction, drug abuse, drug habituation, drug dependence, withdrawal syndrome and overdose.

The pharmaceutical compositions utilized in this invention may be administered by any number of routes including, but not limited to, oral, intravenous, intramuscular, intraarterial, intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, Pa.).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

More particularly, the invention relates to a pharmaceutical composition intended for oral administration.

Pharmaceutical compositions suitable for use in the invention include compositions wherein compounds for depleting mast cells, such as tyrosine kinase inhibitors and c-kit inhibitors, are contained in an effective amount to achieve the intended purpose. The determination of an effective dose is well within the capability of those skilled in the art. A therapeutically effective dose refers to that amount of active ingredient, which ameliorates the symptoms or condition. Therapeutic efficacy and toxicity may be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions which exhibit large therapeutic indices are preferred. As mentioned above, a tyrosine kinase inhibitor and more particularly a c-kit inhibitor according to the invention is unable to promote death of IL-3 dependent cells cultured in presence of IL-3.

## Claims

1. Use of a c-kit inhibitor selected from the group consisting of N-phenyl-2-pyrimidine-amine derivatives having the formula II: 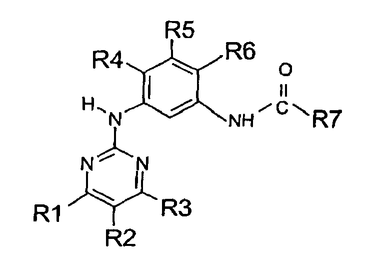 Wherein R1, R2 and R3 are independently chosen from H, F, Cl, Br, I, a C1-C5 alkyl or a cyclic or heterocyclic group, especially a pyridyl group;
R4, R5 and R6 are independently chosen from H, F, Cl, Br, I, a C1-C5 alkyl, especially a methyl group;
and R7 is a phenyl group bearing at least one substituent, which in turn possesses at least one basic site, such as an amino function,
for preparing a medicament for treating substance use disorders.

2. The use according to claim 1, wherein R7 is

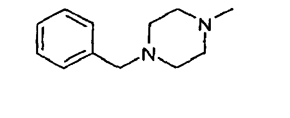

3. The use according to claim 1, wherein said inhibitor is the 4-(4-methylpiperazin-1-ylmethyl)-N-[4-methyl-3-(4-pyridine-3-yl)pyrimidine-2 ylamino)phenyl]-benzamide.

4. The use according to one of claims 1 to 3 for preventing and/or treating substance use disorders in human, more particularly drug addiction, drug abuse, drug habituation, drug dependence, withdrawal syndrome and overdose.

5. The use according to one of claims 1 to 4 wherein said drug is selected from the group consisting of alcohol, nicotine, opioids, cocaine, heroin, anxiolytics and hypnotics such as benzodiazepine, methaqualone and barbiturates, cannabinoids (tetrahydrocannabinol, cannabigerol, cannabinol cannabichromene, cannabidiol, cannabinoid acids), amphetamine such ecstasy, hallucinogen such as LSD, phencyclidine (PCP), mescaline, volatile solvent and volatile nitrites.

## Patentansprüche

1. Verwendung eines c-Kit-Hemmers, ausgewählt aus der Gruppe bestehend aus N-Phenyl-2-pyrimidinamin-Derivaten mit der Formel II: 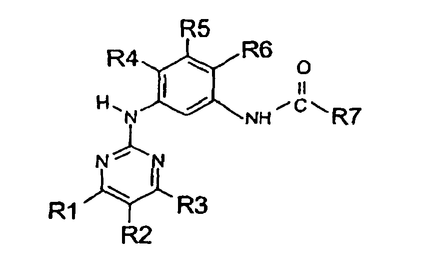 wobei, R1, R2 und R3 unabhängig ausgewählt sind aus H, F, Cl, Br, I und einem C1-C5-Alkyl oder einer cyclischen oder heterocyclischen Gruppe, insbesondere einer Pyridylgruppe;
R4, R5 und R6 unabhängig ausgewählt sind aus H, F, Cl, Br, I und einem C1-C5-Alkyl, insbesondere einer Methylgruppe; und
R7 eine Phenylgruppe ist, die wenigstens einen Substituenten aufweist, der wiederum wenigstens eine basische Stelle besitzt, wie beispielsweise eine Aminofunktion,
zur Herstellung eines Arzneimittels zur Behandlung von Erkrankungen im Zusammenhang mit Substanzgebrauch.

2. Verwendung nach Anspruch 1, wobei R7 ist:

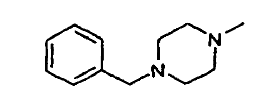

3. Verwendung nach Anspruch 1, wobei der Hemmer das 4-(4-Methylpiperazin-1-ylmethyl)-N-[4-methyl-3-(4-pyridin-3-yl)pyrimidin-2 ylamino)phenyl]-benzamid ist.

4. Verwendung nach einem der Ansprüche 1 bis 3 zum Verhindern und/oder Behandeln von Erkrankungen beim Menschen im Zusammenhang mit Substanzgebrauch, insbesondere Drogensucht, Drogenmissbrauch, Drogengewöhnung, Drogenabhängigkeit, Entzugssyndrom und Überdosis.

5. Verwendung nach einem der Ansprüche 1 bis 4, wobei die Droge ausgewählt ist aus der Gruppe bestehend aus Alkohol, Nikotin, Opioiden, Kokain, Heroin, Anxiolytika und Hypnotika, wie beispielsweise Benzodiazepin, Methaqualon und Barbituraten, Cannabinoiden (Tetrahydrocannabinol, Cannabigerol, Cannabinol Cannabichromen, Cannabidiol, Cannabinoidsäuren), Amphetamin, wie beispielsweise Ecstasy, Halluzinogenen, wie beispielsweise LSD, Phencyclidin (PCP), Meskalin, flüchtigem Lösungsmittel und flüchtigen Nitriten.

## Revendications

1. Utilisation d'un inhibiteur de c-kit choisi dans le groupe constitué par les dérivés de N-phényl-2-pyrimidine-amine de formule II: 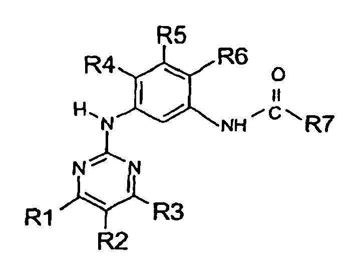 dans laquelle R1, R2 et R3 sont indépendamment choisis parmi H, F, Cl, Br, I, un groupe alkyle en C1-C5 ou un groupe cyclique ou hétérocyclique, en particulier un groupe pyridyle ;
R4, R5 et R6 sont indépendamment choisis parmi H, F, Cl, Br, I, un groupe alkyle en C1-C5, en particulier un groupe méthyle ; et
R7 est un groupe phényle portant au moins un substituant, qui à son tour possède au moins un site basique, tel qu'une fonction amino,
pour la préparation d'un médicament destiné au traitement de troubles liés à l'usage de stupéfiants.

2. Utilisation selon la revendication 1, dans laquelle R7 est

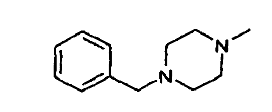

3. Utilisation selon la revendication 1, dans laquelle ledit inhibiteur est le 4-(4-méthylpipérazin-1-ylméthyl)-N-[4-méthyl-3-(4-pyridin-3-yl)pyrimidin-2-ylamino)phényl]-benzamide.

4. Utilisation selon l'une des revendications 1 à 3, pour prévenir et/ou traiter des troubles liés à l'utilisation de substances chez les humains, plus particulièrement la dépendance à une substance médicamenteuse, la toxicomanie, l'accoutumance, la pharmacodépendance, le syndrome de sevrage et le surdosage.

5. Utilisation selon l'une des revendications 1 à 4, dans laquelle ladite substance médicamenteuse est choisie dans le groupe constitué par l'alcool, la nicotine, les opioïdes, la cocaïne, l'héroïne, les anxiolytiques et hypnotiques tels que le benzodiazépine, la méthaqualone et les barbituriques, les cannabinoïdes (tétrahydrocannabinol, cannabigérol, cannabinol, cannabichromène, cannabidiol, acides cannabinoïdes), les amphétamines telles que l'ecstasy, les hallucinogènes tels que le LSD, la phencyclidine (PCP), la mescaline, les solvants volatils et les nitrites volatils.
